# EUROPÄISCHE PATENTSCHRIFT

(11) **EP 1 682 515 B1**
(45) Veröffentlichungstag und Bekanntmachung des Hinweises auf die Patenterteilung: **01.10.2014**
(21) Anmeldenummer: 04765988.3
(22) Anmeldetag: 19.10.2004
(51) Int. Cl.: C07D 231/14, C07D 263/34, C07D 277/30

(54) **VERFAHREN ZUM HERSTELLEN VON FLUORMETHYL-SUBSTITUIERTEN HETEROCYCLEN**
METHOD FOR THE PRODUCTION OF FLUOROMETHYL-SUBSTITUTED HETEROCYCLES
PROCÉDÉ DE FABRICATION D'HÉTEROCYCLES PORTANT DES SUBSTITUANTS FLUOROMÉTHYLES

(30) Priorität: 31.10.2003 DE 10351088
(43) Veröffentlichungstag der Anmeldung: 26.07.2006
(73) Patentinhaber: Bayer CropScience AG, 40789 Monheim (DE)
(72) Erfinder: LANTZSCH, Reinhard, 42115 Wuppertal (DE); PAZENOK, Sergiy, 42699 Solingen (DE); MEMMEL, Frank, 67583 Guntersblum (DE)
(74) Vertreter: BIP Patents
(86) Internationale Anmeldenummer: PCT/EP2004/011809
(87) Internationale Veröffentlichungsnummer: WO 2005/044804

(56) Entgegenhaltungen:
- EP-A1- 0 589 301
- EP-A1- 1 072 576
- WO-A-03/070705
- WO-A1-93/11117
- WO-A1-03/070705
- DE-A- 3 934 924
- US-A- 5 093 347
- US-A- 5 675 016
- US-B1- 6 319 940

## Beschreibung

Die vorliegende Erfindung betrifft ein neues Verfahren zum Herstellen von fluormethyl-substituierten Heterocyclen durch Umsetzung der entsprechenden chlormethyl-substituierten Verbindung mit einem. Fluorierungsmittel.

Es ist bereits bekannt, dass man 3-(Difluormethyl)- bzw. 3-(Fluordichlormethyl)-1-methyl-1H-pyrazol-4-carbonsäureester erhalten kann, indem man das entsprechend halogenierte -2-(Ethoxymethylen)-methylacetoacetat mit Methylhydrazin umsetzt (vgl. WO 92/12970 und WO 93/11117).

Weiterhin ist bekannt, dass man 5-Chlorpyrazol-4-carboxaldehyd-Derivate mit Kaliumfluorid in Dimethylformamid zu der entsprechenden 5-Fluor-Verbindung umsetzen kann (vgl. WO 93/11117).

Die Umwandlung von Heterocyclen, die durch Mono-, Di- oder Trichlormethyl substituiert sind, in fluorierte Analoga ist bisher nur für Pyridinderivate bekannt. Da chlorierte Vorprodukte zur Herstellung von Heterocyclen häufig leichter zugänglich sind als die entsprechenden Fluorverbindungen, besteht Bedarf an einem Verfahren, das es erlaubt, chlorierte Heterocyclen direkt zu fluorierten.

Gegenstand der vorliegenden Erfindung ist also ein Verfahren zum Herstellen von fluormethyl-substituierten Heterocyclen der Formel (I) 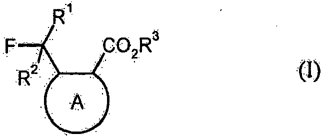 in welcher
- R¹: für, Fluor oder Chlor steht,
- R²: für Wasserstoff, Chlor steht,
- R³: für C₁-C₆-Alkyl steht,
- A: für den 5-gliedrigen Heterocyclus 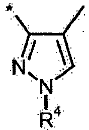 steht, wobei die mit * markierte Bindung mit der -CCIR¹R²-Gruppe und die andere Bindung mit der Estergruppe verbunden ist,
- R⁴: für C₁-C₄-Alkyl, C₃-C₆-Cycloalkyl, C₁-C₄-Alkylthio-C₁-C₄-alkyl, C₁-C₄-Alkoxy-C₁-C₄-alkyl oder Phenyl steht,
dadurch gekennzeichnet, dass man
a) chlormethyl-substituierte Heterocyclen der Formel (II) 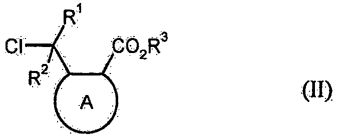 in welcher R¹, R², R³ und A die oben angegebenen Bedeutungen haben,
   in Gegenwart eines Fluorierungsmittels ausgewählt aus 3 HF /N(Et)₃ (Franz Reagenz) und 3 HF/N(n-Bu)₃ und gegebenenfalls in Gegenwart eines Verdünnungsmittels umsetzt.

Überraschenderweise lassen sich die fluormethyl-substituierten Heterocyclen der Formel (I) unter den erfindungsgemäßen Bedingungen mit guten Ausbeuten in hoher Reinheit und Selektivität herstellen. Die Herstellung von Trifluormethyl-, Difluormethyl- oder Monofluormethyl-substituierten aromatischen Verbindungen aus den entsprechend chlorierten Verbindungen durch Halogenaustausch ist für Phenyl- und einige 3-Trihalogenalkyl-pyridin-Derivate zwar bekannt, doch müssen bei diesen Verfahren drastische Bedingungen wie der Einsatz von HF, hohen Temperaturen und Druck angewendet werden. Unter diesen Reaktionsbedingungen würde der Fachmann im Fall von Pyrazol-, Thiazol- und anderen heterocyclischen Verbindungen, die durch Trichlormethyl oder durch das empfindlichere und weniger stabile Dichlormethyl substituiert sind, die Zersetzung des Heterocyclus erwarten (z.B. durch Polymerisation, Hydrolyse oder die Bildung von Säuren und Aldehyden). Dieser Zersetzungsprozess wird sogar beschleunigt, wenn z.B. eine Dichlormethyl-Gruppe direkt mit einem Elekttortenpaar eines Stickstoffatoms konjugiert ist, wie das bei 3-Halogenalkyl-substituierten Pyrazolen der Fall ist. Außerdem ist die Fluorierung von Verbindungen mit einem Substituenten in ortho-Position, insbesondere wenn es sich dabei um die räumlich große und elektronenziehende Carbonsäureester-Gruppe handelt, aus sterischen und elektronischen Gründen deutlich schwieriger durchzuführen. Denn unter stark sauren Bedingungen (z.B. beim Einsatz von HF oder dessen Derivaten) wird die Carbonsäureester-Gruppe normalerweise verseift und bildet einen Ring mit der benachbarten Trichlormethyl- oder Dichlormethyl-Gruppe. Um so überraschender ist es, dass mit dem erfindungsgemäßen Verfahren die fluormethyl-substituierten Heterocyclen der Formel (I) in guten Ausbeuten erhalten werden können.

Verwendet man beispielsweise 3-(Dichlormethyl)-1-methyl-1H-pyrazol-4-carbonsäureethylester als Ausgangsstoff und Triethylamin(tris)hydrofluorid als Fluorierungsmittel, kann das erfindungsgemäße Verfahren (a) durch das folgende Formelschema veranschaulicht werden.

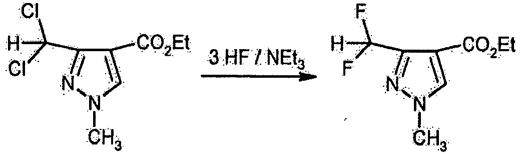

Die bei der Durchführung des erfindungsgemäßen Verfahrens als Ausgangsstoffe verwendeten chlormethyl-substituierte Heterocyclen sind durch die Formel (II) allgemein definiert. Die Substituenten haben folgende bevorzugte Bedeutungen:
- R¹: steht für Fluor oder Chlor.
- R²: steht für Wasserstoff, Fluor oder Chlor.
- R³: steht bevorzugt für C₁-C₄-Alkyl, besonders bevorzugt für Methyl, Ethyl, n-Propyl, iso-Propyl, n-Butyl, sec-Butyl, iso-Butyl oder tert-Butyl, ganz besonders bevorzugt für Methyl oder Ethyl.
- A: für den 5-gliedrigen Heterocyclus 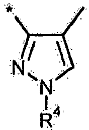 steht,
wobei jeweils die mit * markierte Bindung mit der -CCIR¹R²-Gruppe und die andere Bindung mit der Estergruppe verbunden ist,
- R⁴: steht bevorzugt für Methyl, Ethyl, n-Propyl, iso-Propyl, Cyclopropyl, Cyclopentyl, Cyclohexyl oder Phenyl, besonderes bevorzugt für Methyl, Ethyl, iso-Propyl oder Phenyl, ganz besonders bevorzugt für Methyl.

Bevorzugt werden chlormethyl-substituierte Heterocyclen der Formel (II) als Ausgangsstoff eingesetzt, in welcher R¹ für Chlor und R² für Wasserstoff steht.

Als Ausgangsstoffe der Formel (II) sind besonders bevorzugt chlomethyl-substituierte Heterocyclen der Formel (II-a) 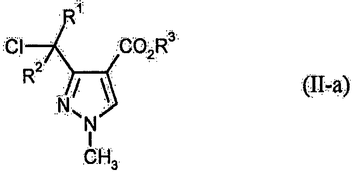 in welcher R¹, R² und R³ die oben angegebenen Bedeutungen haben.

Ganz besonders bevorzugt sind Verbindungen der Formen (II-a), in welcher R¹ für Chlor, R² für Wasserstoff und R³ für Methyl oder Ethyl steht.

Chlormethyl-substituierte Heterocyclen der Formel (II) sind teilweise bekannt (vgl. WO 92/12970, WO 93/11117 und The Chemistry of Heterocyclic Compounds: Thiazole and its derivatives, Jaques Metzger (ed.), Vol. 34, Part 1-3, John Wiley and Sons, New York, 1979).

3-Halogenmethyl-1H-pyrazol-4-carbonsäureester lassen sich herstellen, indem man
b) Säurehalogenide der Formel (III) 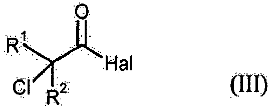 in welcher
   R¹ und R² die oben angegebenen Bedeutungen haben,
   Hal für Fluor, Chlor oder Brom steht,
   mit Dialkylaminoacrylsäureestern der Formel (IV) 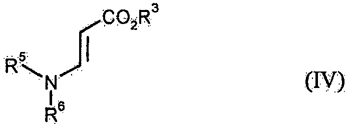 in welcher
   R³ die oben angegebenen Bedeutungen hat,
   R⁵ und R⁶ unabhängig voneinander für C₁-C₄-Alkyl stehen,
   in einem mit Wasser nicht mischbaren organischen Lösungsmittel (z.B. Toluol) in Gegenwart einer Base (z.B. Natriumhydroxid oder Pyridin) umsetzt,
   und die so erhaltenen 2-Dihalogenacyl-3-amino-acrylsäureester der Formel (V) 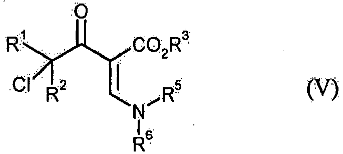 in welcher R¹, R², R³, R⁵ und R⁶ die oben angegebenen Bedeutungen haben, mit Hydrazin-Derivaten der Formel (VI)

   R⁴-NH-NH₂ (VI)

   in welcher R⁴ die oben angegebenen Bedeutungen halt,
   in Gegenwart eines Verdünnungsmittels (z.B. Toluol) umsetzt.

Das erfindungsgemäße Verfahren (a) wird in Gegenwart eines Fluorierungsmittels durchgeführt. Hierzu kommen alle für solche Reaktionen üblichen Fluorierungsmittel infrage. Vorzugsweise verwendbar sind z.B. Alkalifluoride wie Natriumfluorid, Kaliumfluorid und Caesiumfluorid, Cobalt(III)-fluorid, Halogenfluoride, Antimonfluoride, Molybdänfluorid, Fluorwasserstoff Fluorwasserstoff/ Pyridin-Gemische, tertiäre Ammoniumhydrofluoride oder Trialkylaminhydrofluoride der allgemeinen Formen n HF /N(Alk)₃ (wobei n für 1, 2, oder 3, bevorzugt für 2 oder 3, und Alk für C₁-C₄-Alkyl, bevorzugt für Ethyl oder n-Butyl, steht). Besonders bevorzugt verwendet man 3 HF/N(Ft)₃ (Franz Reagenz), 3HF/N(n-Bu)3 und HF/Pytidin (Olah's Reagenz). Ganz besonders bevorzugt verwendet man 3 HF /N(Et)₃ (Franz Reagenz) oder 3 HF/N(n-Bu)₃.

Das erfindungsgemäße Verfahren wird gegebenenfalls in Anwesenheit eines Verdünnungsmittels durchgerührt. Vorzugsweise verwendbar sind Nitrile, wie Acetonitril; halogenierte Kohlenwasserstoffe, wie z.B. Chlorbenzol, Dichlorbenzol, Dichlorethan, Chloroform, Tetrachlormethan, Dichlorethan, Trifluorchlormethan, 1,1,2-Trifluor-1,2,2-trichlorethan, 1,1,1-Trifluor-2,2,2-trichlorethan oder Trichloroethan, aromatische Kohlenwasserstoffe, wie z.B. Petrolether, Hexan, Heptan, Cyclohexan, Methylcyclohexan, Benzol, Toluol, Xylol oder Decalin; Ether, wie Diethylether, Diisopropylether, Methyl-tert-butylether, Methyl-tert-Amylether, Dioxan, Tetrahydrofuran, 1,2- Dimethoxyethan, 1,2-Dimethoxyethan oder Anisol; Diethylenglykol. Besonders bevorzugt verwendet man Acetonitril, Toluoyl, Chlorbenzol, Trifluorchlormethan, 1,1,2-Trifluor-1,2,2-trichlorethan, 1,1,1-Trifluor-2,2,2-trichlorethan, Dioxan oder Diethylenglykol.

Bei der Durchführung des erfindungsgemäßen Verfahrens kann innerhalb eines relativ großen Temperaturbereichs gearbeitet werden. Im Allgemeinen arbeitet man bei Temperaturen von 80°C bis 170°C oder im Bereich von 20°C bis 170°C, bevorzugt bei Temperaturen von 120°C bis 150°C oder 100°C bis 150°C.

Das erfindungsgemäße Verfahren wird im Allgemeinen unter Normaldruck durchgeführt. Es ist jedoch auch möglich, unter erhöhtem oder vermindertem Druck - im Allgemeinen zwischen 0,1 bar und 50 bar, bevorzugt zwischen 1 bar und 10 bar- zu arbeiten.

Die Reaktionszeit ist nicht kritisch und kann in Abhängigkeit von der Ansatzgröße in einem größeren Bereich von 1 h bis 20 h, bevorzugt von 6 h bis 12 h gewählt werden.

Bei der Durchführung des erfindungsgemäßen Verfahrens setzt man auf 1 Mol chlormethyl-substituierte Heterocyclen der Formel (II) im Allgemeinen zwischen 1 Mol und 3 Mol an gebundenem HF, vorzugsweise zwischen 1 Mol und 1.5 Mol je Chloratom an Fluorierungsmittel ein.

Die durch das erfindungsgemäßen Verfahren herstellbaren fluormethyl-substituierten Heterocyclen der Formel (I) sind wertvolle Vorstufen für die Herstellung von halogenmethyl-substituierten Pyrazolyl-, Thiazolyl- und Oxazolylcarboxamide, welche fungizide Wirkstoffe darstellen (vgl. WO 03/070705).

So werden z.B. fungizid wirksame Carboxamide der Formel (VII) 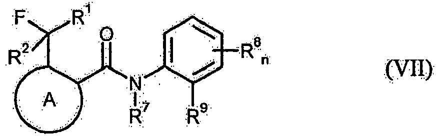 in welcher
- R¹, R² und A: die oben angegebenen Bedeutungen haben,
- R⁷: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Haloenalkylthio, C₁-C₄-Halogen-alkylsulfonyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formel-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alko-xy)-carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor-und/oder Bromatomen; oder -C(=O)C(=O)R¹⁰, -CONR¹¹R¹² oder -CH₂NR¹³R¹⁴ steht,
- R⁸: für Wasserstoff, Fluor, Chlor, Methyl, iso-Propyl, Methylthio oder Trifluormethyl steht,
- n: für 1, 2, 3 oder 4, bevorzugt für 1 oder 2 steht,
- R⁹: für gegebenenfalls einfach bis fünffach, gleich oder verschieden substituiertes Phenyl steht, wobei die Substituenten ausgewählt sind aus Halogen_{;} C₁-C₄-Alkyl, C₁-C₄-Alkoxy, C₁-C₂-Halogenalkyl oder C₁-C₂-Halogenalkoxy mit jeweils 1 bis 5 Fluor-, Chlor- und/oder Bromatomen, Hydroxyimino-C₁-C₄-alkyl, C₁-C₄-Alkoxyimino-C₁-C₄-alkyl, C₁-C₄-Halogenalkoxyimino-C₁-C₄-alkyl, oder bei zwei benachbarten Substituenten aus Difluormethylendioxy oder Tetrafluorethylendioxy;
oder für jeweils gegebenenfalls einfach bis vierfach, gleich oder verschieden durch Halogen und/oder C₁-C₄-Alkyl substituiertes C₃-C₁₀-Cycloalkyl oder C₃-C₁₀-Bicycloalkyl;
oder unsubstituiertes C₂-C₂₀-Alkyl oder für einfach oder mehrfach, gleich oder verschieden durch Fluor, Chlor, Brom, Iod und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann;
oder für jeweils gegebenenfalls einfach oder mehrfach, gleich oder verschieden durch Fluor, Chlor, Brom, Iod und/oder C₃-C₆-Cycloalkyl substituiertes C₂-C₂₀-Alkenyl oder C₂-C₂₀-Alkinol steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann;
- R¹⁰: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R¹¹ und R¹²: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R¹¹ und R¹²: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹⁵ enthalten kann,
- R¹³ und R¹⁴: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R¹³ und R¹⁴: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹⁵ enthalten kann,
- R¹⁵: für Wasserstoff oder C₁-C₆-Alkyl steht,
erhalten, indem man fluormethyl-substituierte Heterocyclen der Formel (I) 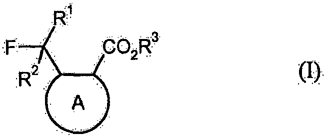 in welcher R¹, R², R³ und die oben angegebenen Bedeutungen haben,
in Anwesenheit einer Base und gegebenenfalls in Gegenwart eines Verdünnungsmittels verseift und die freie Säure anschließend entweder in Gegenwart eines Chlorierungsmittels und gegebenenfalls in Gegenwart eines Verdünnungsmittels in das entsprechende Säurechlorid überführt oder die freie Säure direkt mit Anilin-Derivaten der Formel (VIII) 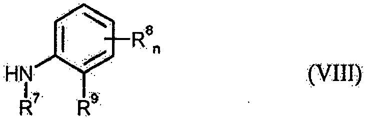 in weicher R⁷, R⁸, n und R⁹ die oben angegebenen Bedeutungen haben,
gegebenenfalls in Gegenwart eines Katalysators, gegebenenfalls in Gegenwart eines Kordensationsmittels, gegebenenfalls in Gegenwart eines Säurebindemittels und gegebenenfalls in Gegenwart eines Verdünnungsmittels umsetzt.

Das erfindungsgemäße Herstellen von fluormethyl-substituierten Hetercyclen der Formel (I) wird in den nachstehenden Beispielen beschrieben, welche die obige Beschreibung weiter illustrieren.

### Herstellungsbeispiele

### Beispiel 1

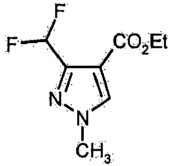

3-(Dichlormethyl)-1-methyl-1H-pyrazol-4-carbonsäureethylester (37.9 g, 0.16mol) und Triethylammtris(hydrofluorid) (80 g, 0.49 mol) wurden in einem Autoklav 8 h bei 145°C erhitzt. Das Reaktionsgemisch wurde dann mit 200 ml Wasser verdünnt. Das ausgefallene Produkt wurde abfiltriert, gewaschen und getrocknet.

Man erhielt 28.5 g (87 % der Theorie) von 3-(Difluormethyl)-1-methyl-1H-pyrazol-4-carbonsäureethylester Produkt mit dem Schmelzpunkt 43-46°C.
¹H-NMR (CDCl₃): δ = 1.35 (t, 3H); 3.96 (t, 3H); 4.31 (q, CH₂); 7.10 (t, CF₂H); 7.90 (s, 1H) ppm. ¹⁹F-NMR (CDCl₃): δ =-117.2 (d , J = 55.4 Hz) ppm.

### Beispiel 2

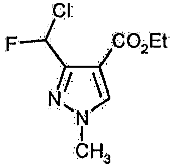

3-(Dichlormethyl)-1-methyl-1H-pyrazol-4-carbonsäureethylester 37.9g (0.16 mol) und Triethyl-amintris(hydrofluorid) 80 g (0.49 mol) wurden in einem Autoktav 8 h bei 120°C erhitzt. Das Reaktionsgemisch wurde dann mit 200 ml Wasser verdünnt und das Produkt mit Ethylacetat extrahiert. Das gewünschte Produkt wurde durch Chromatographie von 3-(Difluormethyl)-1-methyl-1H-pyrazol-4-carbonsäureethylester abgetrennt und isoliert.

Man erhielt 20 g (56 % der Theorie) von 3-(Fluorchlormethyl)-1-methyl-1H-pyrazol-4-carbonsäureethylester.
¹⁹F-NMR (CDCl₃): δ 133.8 (d , J= 47.5 Hz) ppm.

### Beispiel 3 (nicht erfindungsgemäß)

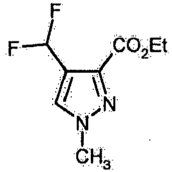

4-(Dichlormethyl)-1-methyl-1H-pyrazol-3-carbonsäureethylester 37.9g (0.16 mol) und Triethyl-aminti-is(hydrofluorid) 80 g (0.49 mol) wurden in einem Atiroklav 8 h bei 160°C erhitzt. Das Reaktionsgemisch wurde dann mit 200 ml Wasser verdünnt und das Produkt mit Ethytacetät extrahiert. Das gewünschte Produkt wurde durch Chromatographie gereinigt und isoliert.

Man erhielt 26.8 g (75% der Theorie) von 4-(Difluormethyl)-1-methyl-1H-pyrazol-3-carbonsäureethylester mit dem Schmelzpunkt 30-31°C.
¹H-NMR (CDCl₃): δ = 1.37 (t, 3H); 4.07 (t, 3H); 4.31 (q, CH₂); 7.49 (t, CF₂H); 7.86 (s, 1H) ppm, ¹⁹F-NMR (CDCl₃): δ = -117.2 (d,J = 54.7 Hz) ppm.

## Patentansprüche

1. Verfahren zum Herstellen von fluormethyl-substituierten Heterocyclen der Formel (I) 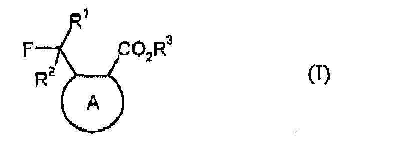 in welcher
R¹ für Fluor oder Chlor steht,
R² für Wasserstoff steht,
R³ für C₁-C₆-Alkyl steht,
A für den 5-gliedrigen Heterocyclus 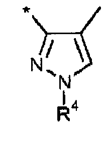 steht,
wobei die mit * markierte Bindung mit der -CCIR¹R²-Gruppe und die andere Bindung mit der Estergruppe verbunden ist,
R⁴ für C₁-C₄-Alk_{Y}1, C₃-C₆-Cycloalkyl, C₁-C₄-Alkylthio-C₁-C₄-alkyl, C₁-C₄-Alkoxy-C₁-C₄-alkyl oder Phenyl steht,
**dadurch gekennzeichnet, dass** man
a) chlormethyl-substituierte Heterocyclen der Formel (II) 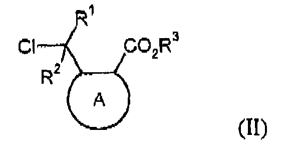 in welcher R¹, R², R³ und A die oben angegebenen Bedeutungen haben,
in Gegenwart eines Fluorierungsmittels ausgewählt aus 3 HF/N(Et)₃ (Franz Reagenz) und 3 HF / N(n-Bu)₃ und gegebenenfalls in Gegenwart eines Verdünnungsmittels umsetzt.

2. Verfahren gemäß Anspruch 1, **dadurch gekennzeichnet, dass** man als Ausgangsstoffe chlormethyl-substituierte Heterocyclen der Formel (II) einsetzt, in welcher
R¹ für Chlor steht, und
R², R³, A und
R⁴ die in Anspruch 1 angegebenen Bedeutungen haben.

3. Verfahren gemäß einem der Anspruche 1 oder 2, **dadurch gekennzeichnet, dass** man als Ausgangsstoffe die folgende Verbindung der Formel (II-a) einsetzt 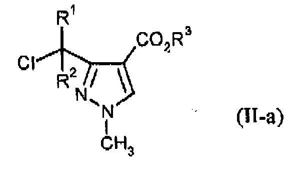 in welcher R¹, R² und R³ die in Anspruch 1 oder 2 angegebenen Bedeutungen haben.

4. Verfahren gemäß Anspruch 3, **dadurch gekennzeichnet, dass** R¹ für Chlor, R² für Wasserstoff und R³ für Methyl oder Ethyl steht.

5. Verfahren gemäß einem oder mehreren der Ansprüche 1 bis 4, **dadurch gekennzeichnet, dass** man bei Temperaturen von 80°C bis 170°C durchfuhrt.

6. Verfahren gemäß einem oder mehreren der Ansprüche 1 bis 5, **dadurch gekennzeichnet, dass** man bei Temperaturen von 120°C bis 150°C durchführt.

7. Verfahren gemäß Anspruch 1 zum Herstellen des fluormethyl-substituierten Heterozyklus der Formel (Ia) 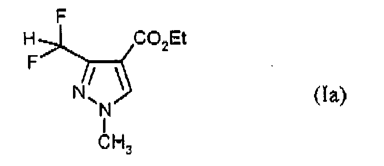 **dadurch gekennzeichnet dass** man
a) den Chlormethyl-substitutierten Heterozyklus der Formel (IIb) 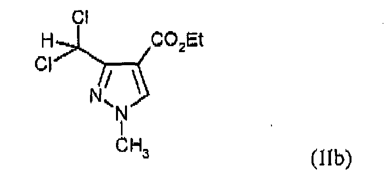 in Gegenwart des Fluorierungsmittels 3 HF /N(Et)₃ (Franz Reagenz) und gegebenenfalls in Gegenwart eines Verdünnungsmittels umsetzt.

## Claims

1. Process for preparing fluoromethyl-substituted heterocycles of the formula (I) 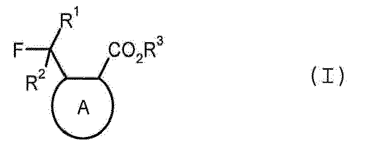 in which
R¹ is fluorine or chlorine,
R² is hydrogen,
R³ is C₁-C₆-alkyl,
A is the 5-membered heterocycle 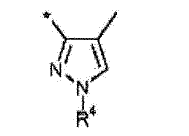 where the bond marked by * is joined to the -CClR¹R² group and the other bond to the ester group,
R⁴ is C₁-C₄-alkyl, C₃-C₆-cycloalkyl, C₁-C₄-alkylthio-C₁-C₄-alkyl, C₁-C₄-alkoxy-C₁-C₄-alkyl or phenyl,
**characterized in that**
a) chloromethyl-substituted heterocycles of the formula (II) 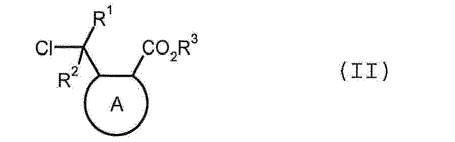 in which R¹, R², R³ and A are each as defined above
are converted in the presence of a fluorinating agent selected from 3 HF / N(Et)₃ (Franz reagent) and 3 HF / N(n-Bu)₃, and optionally in the presence of a diluent.

2. Process according to Claim 1, **characterized in that** the starting materials used are chloromethyl-substituted heterocycles of the formula (II),
in which
R¹ is chlorine, and
R², R³, A and R⁴ are as defined in Claim 1.

3. Process according to one of Claims 1 and 2, **characterized in that** the starting materials used are the following compound of the formula (II-a) 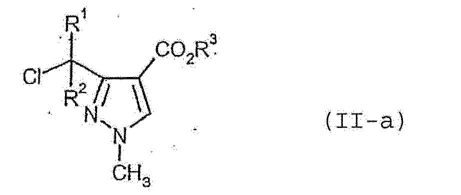 in which R¹, R² and R³ are as defined in Claim 1 or 2.

4. Process according to Claim 3, **characterized in that** R¹ is chlorine, R² is hydrogen and R³ is methyl or ethyl.

5. Process according to one or more of Claims 1 to 4, **characterized in that** it is carried out at temperatures of 80°C to 170°C.

6. Process according to one or more of Claims 1 to 5, **characterized in that** it is carried out at temperatures of 120°C to 150°C.

7. Process according to Claim 1 for preparing the fluoromethyl-substituted heterocycle of the formula (Ia) 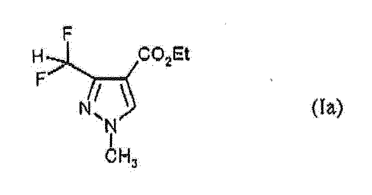 **characterized in that**
a) the chloromethyl-substituted heterocycle of the formula (IIb) 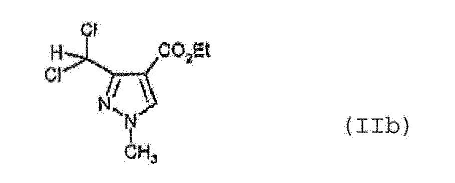
is converted in the presence of the fluorinating agent 3HF/N(Et)₃ (Franz reagent) and optionally in the presence of a diluent.

## Revendications

1. Procédé de fabrication d'hétérocycles à substitution fluorométhyle de formule (I) 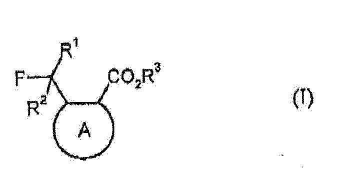 dans laquelle
R¹ représente fluor ou chlore,
R² représente hydrogène,
R³ représente alkyle en C₁-C₆,
A représente l'hétérocycle à 5 éléments 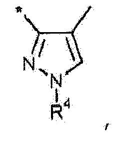
la liaison marquée avec * étant reliée avec le groupe - CClR¹R² et l'autre liaison avec le groupe ester,
R⁴ représente alkyle en C₁-C₄, cycloalkyle en C₃-C₆, alkylthio en C₁-C₄-alkyle en C₁-C₄, alcoxy en C₁-C₄-alkyle en C₁-C₄ ou phényle,
**caractérisé en ce que**
a) des hétérocycles à substitution chlorométhyle de formule (II) 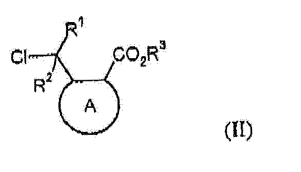
dans laquelle R¹-, R², R³ et A ont les significations indiquées précédemment,
sont mis en réaction en présence d'un agent de fluoration choisi parmi 3 HF/N(Et)₃ (réactif de Franz) et 3 HF/N(n-Bu)₃ et éventuellement en présence d'un diluant.

2. Procédé selon la revendication 1, **caractérisé en ce que** des hétérocycles à substitution chlorométhyle de formule (II), dans laquelle
R¹ représente chlore et
R², R³, A et R⁴ ont les significations indiquées dans la revendication 1,
sont utilisés en tant que matières premières.

3. Procédé selon l'une quelconque des revendications 1 ou 2, **caractérisé en ce que** le composé de formule (II-a) suivant est utilisé en tant que matière première 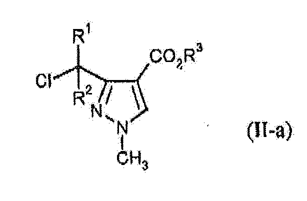 dans laquelle R¹, R² et R³ ont les significations indiquées dans la revendication 1 ou 2.

4. Procédé selon la revendication 3, **caractérisé en ce que** R¹ représente chlore, R² représente hydrogène et R³ représente méthyle ou éthyle.

5. Procédé selon une ou plusieurs des revendications 1 à 4, **caractérisé en ce qu'**il est réalisé à des températures de 80 °C à 170 °C.

6. Procédé selon une ou plusieurs des revendications 1 à 5, **caractérisé en ce qu'**il est réalisé à des températures de 120 °C à 150 °C.

7. Procédé selon la revendication 1, pour la fabrication de l'hétérocycle à substitution fluorométhyle de formule (Ia) 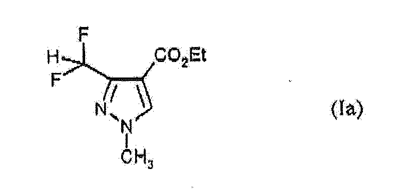 **caractérisé en ce que**
a) l'hétérocycle à substitution chlorométhyle de formule (IIb) 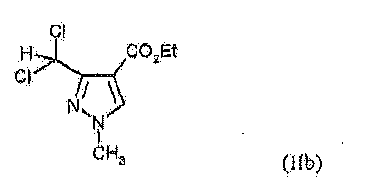 est mis en réaction en présence de l'agent de fluoration 3 HF/N(Et)₃ (réactif de Franz) et éventuellement en présence d'un diluant.
